**Europäisches Patentamt**

**European Patent Office**

**Office européen des brevets**

(11) Publication number : **0 234 684 B1**

# (12) EUROPEAN PATENT SPECIFICATION

(45) Date of publication of patent specification :
**10.07.91 Bulletin 91/28**

(51) Int. Cl.⁵ : **C07C 5/27, C07C 15/08**

(21) Application number : **87300162.2**

(22) Date of filing : **08.01.87**

(54) **Xylene isomerization process.**

(30) Priority : **15.01.86 US 819072**

(43) Date of publication of application :
**02.09.87 Bulletin 87/36**

(45) Publication of the grant of the patent :
**10.07.91 Bulletin 91/28**

(84) Designated Contracting States :
**BE DE FR GB IT NL**

(56) References cited :
**GB-A- 1 583 707**
**US-A- 4 117 026**
**US-E- 31 782**

(73) Proprietor : **MOBIL OIL CORPORATION**
**150 East 42nd Street**
**New York New York 10017 (US)**

(72) Inventor : **LaPierre, Rene Bernard**
**43 Arrowhead Court**
**Medford New Jersey 08055 (US)**
Inventor : **Kresge, Charles Theodore**
**4221 Bandana Way**
**Ellicott City Maryland 21043 (US)**
Inventor : **Chu, Yung-Feng**
**30 Tond View Drive**
**Plainsboro New Jersey 08536 (US)**

(74) Representative : **Cooper, John Anthony et al**
**Mobil Court 3 Clements Inn**
**London WC2A 2EB (GB)**

## Description

This invention relates to a xylene isomerization process which is capable of converting ethylbenzene and non-aromatics exhaustively while selectively isomerizing xylenes to thermal equilibrium.

Xylenes are valuable industrial chemicals derived primarily from aromatic naphthas such as petroleum reformates and pyrolysis gasolines. Petroleum reformates result from processing petroleum naphthas over a catalyst such as platinum on alumina at temperatures which favor dehydrogenation of naphthenes. Pyrolysis gasolines are liquid products resulting from steam cracking of hydrocarbons to manufacture ethylene, propylene, and the like.

Generally, regardless of the aromatic naphtha source, it has been the practice to subject the liquid hydrocarbon to extraction with a solvent highly selective for aromatics to obtain an aromatic mixture of the benzene and alkylated benzenes present in the aromatic naphtha. The resulting extract can then be distilled to separate benzene, toluene and $C_8$ aromatics from higher boiling compounds in the extract. Benzene and toluene are recovered in high purity ; however, the $C_8$ fraction, containing valuable para-xylene, is a mixture of three xylene isomers with ethylbenzene. These mixtures will also contain $C_8$-$C_9$ paraffins, the amount of which is determined both by the source of the naphtha as well as the efficiency of the solvent extraction.

As commercial use of para- and ortho-xylene has increased, there has been interest in isomerizing the xylene isomers toward an equilibrium mix and thus increasing yields of the desired xylenes. Of the xylene isomers, meta-xylene is the least desired product, while ortho- and para-xylene are the most desired products. Para-xylene is of particular value as it is useful in the manufacture of terephthalic acid which is an intermediate in the manufacture of polyester and synthetic fibers.

In practice, isomerization processes are operations used in conjunction with xylene separation processes. A virgin $C_8$ aromatics mixture is fed to such a combined process system, along with undesired isomers emerging from the product separation steps. The feed is charged to the isomerizing unit and the effluent isomerizate $C_8$ aromatics are sent to the product separation steps. The composition of isomerizer feed is then a function of the virgin $C_8$ aromatic feed, the product separation unit performance, and the isomerizer performance. The objective in the isomerization reactor is to bring the charge as near to the equilibrium concentration as may be feasible consistent with reaction times which do not give extensive cracking and disproportionation. The thermodynamic equilibrium varies slightly with temperature.

The rate of ethylbenzene conversion in a $C_8$ aromatic mixture is related to effective contact time. Hydrogen partial pressure can also have a very significant effect on ethylbenzene conversion. Products formed from ethylbenzene include $C_8$ naphthenes, benzene from hydrocracking ethylbenzene and $C_9^+$ aromatics from disproportionation, as well as total loss to other than $C_8$ molecular weight components, such as $C_5$ and lighter hydrocarbon byproducts.

By comparison, the three xylene isomers isomerize much more selectively than does ethylbenzene. However, the xylene isomers exhibit different rates of isomerization and hence, with different feed composition situations, the rates of approach to equilibrium vary considerably. Loss of xylenes to other molecular weight products varies with contact time. By-products include naphthenes, toluene, $C_9$ and heavier aromatics and $C_5$ and lighter hydrocracking products.

Because of the deleterious effects of ethylbenzene build up in the loop manufacture of xylenes and because of the great expense of removing it from mixed $C_8$ aromatics, a process which would result in ethylbenzene conversion at a rate approaching that of xylene isomerization would be desirable provided xylene losses can be maintained at a reasonable level. Progress toward such a goal was provided by U.S. Patent No. 4,163,028 which describes an isomerization process conducted at 430-540°C (800 to 1000°F) in the presence of zeolite catalyst having a constraint index of about 1 to 12 and having a silica/alumina ratio of at least 500. An improved catalyst for this process was later described in U.S. Patent No. 4,312,790.

However, even the use of catalysts of U.S. Patent Nos. 4,163,028 and 4,312,790 can result in unacceptably high xylene losses. For example, we have determined that catalysts of U.S. Patent Nos. 4,163,028 and 4,312,790 give unacceptably high xylene losses when xylene isomerization feeds containing greater than 15% ethylbenzene are processed under conditions which give greater than about 50% conversion of ethylbenzene per pass. This situation is further aggravated if the isomerization feed contains paraffins. An object of the present invention is to minimize or overcome these problems.

Accordingly, the invention resides in a process for the treatment of a feed which contains an aromatic $C_8$ hydrocarbon mixture of ethylbenzene and xylenes, in which the concentration of para-xylene is less than that at thermal equilibrium, for dealkylating ethylbenzene and isomerizing xylenes to increase the concentration of para-xylene which process comprises contacting the feed with a two component catalyst system including component (1) and component (2) wherein

component (1) comprises a zeolite having a constraint index of 1 to 12 and an ortho-xylene sorption time of greater than 50 minutes based on its capacity to sorb 30% of the equilibrium capacity of ortho-xylene at 120°C and at an ortho xylene partial pressure of 600 ± 100 Pa (4.5 ± 0.8 mm of mercury) and 0.05 to 10 wt% of a hydrogenation/dehydrogenation component ; and

component (2 comprises a zeolite, having a constraint index of 1 to 12 and a xylene sorption capacity greater than 1 gram/100 grams of zeolite and an ortho-xylene sorption time of less than 10 minutes based on its capacity to sorb 30% of the equilibrium sorption capacity of ortho-xylene at 120°C and an ortho-xylene partial pressure of 600 ± 100 Pa (4.5 ± 0.8 mm of mercury) and comprises 0.05 to 10 weight percent of a hydrogenation/ dehydrogenation component, component (2) comprising at least 50 percent by volume of the catalyst system.

The process of the invention is effective to exhaustively convert ethylbenzene and non-aromatics in a mixed ethylbenzene/xylene feed, while simultaneously converting xylenes to thermal equilibrium concentration. By exhaustively converting ethylbenzene and non-aromatics, we mean that per pass at least half the ethylbenzene is deethylated and at least a third of the non-aromatics, paraffins, are hydrocracked without excessive loss of xylenes to heavier aromatics. In this way, the volume of the recycle stream and/or complexity of the separation processes is minimized.

The present process comprises contacting an isomerization feed containing $C_8$ aromatics and paraffins, with an improved catalyst system, under conversion conditions including a temperature of 200-540°C (400-1000°F), a pressure of 100 to 7000 kPa (0-1000 psig), a WHSV of 0.5 and 100 and a $H_2/H_C$ molar ratio of between about 0.5 and 10. Preferably, those conditions include a temperature of 400-480°C (750 to 900°F), a pressure of 440 to 2860 kPa (50 to 400 psig), a WHSV of 3 to 50 and $H_2/H_C$ molar ratio of 1 to 5.

The catalyst employed is a two component system. One of the two components, component (1), selectively converts ethylbenzene to benzene and ethane and hydrocracks paraffins. The other component, component (2), of the catalyst system isomerizes the xylenes to effect isomerization to the extent that the amount of para-xylene in the isomerization product effluent is at least that at the thermal equilibrium of the xylene(s). In one embodiment of the process, the component of the catalyst system effective for converting the ethylbenzene in the mixed xylenes will show reduced activity for isomerization of the xylenes. Each of the zeolite catalysts contains an amount of a noble metal effective to increase the hydrogenative activity of the zeolite.

Xylene Isomerization Feeds

Xylere isomerization feeds, processed in accordance with the invention, are any aromatic $C_8$ mixture containing ethylbenzene and xylene(s). Generally, such a mixture will have an ethylbenzene content in the range of 5 to 60 weight %, an ortho-xylene content in the range of 0 to 35 weight %, a meta-xylene content in the range of 20 to 95 weight %, and a para-xylene content in the range of 0 to 15 weight %. Other xylene isomerization processes which operate to convert the ethylbenzene in the isomerization feed require high ethylbenzene content in the feed and thus involve ethylbenzene and naphthene recycle to maintain high ethylbenzene content in the isomerization feed. The feed in addition to the above aromatic $C_8$ mixture can contain non-aromatic hydrocarbons, such as paraffins and naphthenes. The paraffins will comprise 0 to 20 weight percent of the feed ; generally, the paraffins will comprise $C_8$-$C_9$ paraffins.

Process Conditions

In the process of the invention, the isomerization feed is contacted with a multi-functional catalyst system under conversion conditions which, as indicated above include a temperature of 200-540°C (400-1000C°F), a pressure of 100 to 7000 kPa (0-1000 psig), a WHSV of 0.5 and 100 and a $H_2/H_C$ molar ratio of 0.5 and 10. Preferably, those conditions include a temperature of 400 to 480°C (750 to 900°F), a pressure of (50 to 400 psig), a WHSV of 3 to 50 and $H_2/H_C$ molar ratio of 1 to 5.

The conversion process may be carried out as a batch type, semi-continuous or continuous operation. Where a moving-bed reactor is used, tie catalyst system can be regenerated in a regeneration zone in which coke is burned from the catalyst in an oxygen containing atmosphere, e.g., air at an elevated temperature after which the regenerated catalyst is recycled to the conversion zone for further contact with the charge stock. In a fixed bed reactor, regeneration can be carried out using initially an inert gas containing a small amount of oxygen (0.5-2%) to burn cake in a controlled manner so as to limit the temperature to a maximum of around 500°-550°C.

In general, the xylene isomerization reaction is carried out in a fixed bed reactor containing the catalyst system described above. In a preferred embodiment the two components of the catalyst system are in sequential beds. That is, the component of the catalyst system used in the process of the invention which is effective to isamerize the xylene components forms one part of the bed, while the other component of the catalyst system forms the remaining portion of the catalyst bed. Thus, in theory, the conversion process could be carried out in two different reactors. Preferably, however, the feed is cascaded over the catalyst system disposed in the reactor in sequential beds. In cascading, the feed is contacted with the two components of the catalyst system without intervening separation of light gases.

In one preferred embodiment, component (1) of the catalyst system effective to deethylate and hydrocrack paraffins is upstream with respect to the catalyst component (2) which is effective to isomerize the xylene components of the $C_8$ aromatic feed. In this embodiment, the catalyst component (1) which is effective to deethylate ethylbenzene is preferably employed in a volume no greater than 1/2, and more preferably no greater than 1/3 of the volume of the catalyst bed. For example, the catalyst component (1) to deethylate ethylbenzene may comprise 25 percent of the bed volume while the xylene isomerization component (2) comprises 75 percent of the bed volume.

After the conversion process the isomerization product can be treated to isolate para-xylene. Thus, the isomerizate product can be fed to a crystallizer to crystallize para-xylene. The residual isomerizate can then be stripped of products lighter than $C_8$ while products heavier than $C_9$ in the residual isomerizate are further processed. $C_8$ fractions from which para-xylene has been removed can be recycled to the isomerizer.

One result of the process of the invention is to convert the mixed xylene components of the feed containing p-xylene in an amount less than that at thermal equilibrium to an extent such that product effluent from the isomerizer contains p-xylene in an amount at least approaching that of p-xylene in the xylene mixture produced at thermal equilibrium. Another result of the process of the invention is exhaustive conversion of ethyl benzene, i.e. at least 50% conversion of ethyl benzene in the feed which is isomerized.

Thus, in accordance with the invention, ethyl benzene conversion during the isomerization surpasses that possible by prior isomerization processes. Moreover, xylene yields are at least comparable and most often greater than those of prior isomerization processes as a result of lower losses to $C_8^+$ aromatics.

## Catalyst Composition

The catalyst composition employed in the present process comprises two components each of which is characterized by two common factors. Thus each component contains a strong hydrogenation/dehydrogenation component and each comprises a zeolite having a Constraint Index (see U.S. Patent No. 4,016,218) when measured at a temperature of 290-538°C within the range 1 to 12. Zeolites having a constraint index within the approximate range of 1 to 12 are often grouped as members of the class of zeolites referred as shape selective, of which the preferred member is ZSM-5.

Generally, the zeolite, either directly or via initial ammonium exchange followed by calcination, is in the hydrogen from such that a predominant proportion of its exchangeable cations are hydrogen ions. In general, it is contemplated that more than 50 percent and preferably more than 75 percent of the cationic sites of the crystalline aluminosilicate zeolite will be occupied by hydrogen ions.

As indicated above each of the two components of the present catalyst system is a zeolite which is associated with a hydrogenation/dehydrogenation component. The hydrogenation/dehydrogenation component is preferably a noble metal such as platinum, palladium, or indium, rhenium and rhodium, with platinum being most preferred. Combinations of noble metals suchs platinum-rhenium, platinum-palladium, platinum-iridium or platinum-iridium-rhenium together with combinations with non-noble metals, particularly of Groups VIA and VIIIA are of interest, particularly with metals such as cobalt, nickel, vanadium, tungsten, titanium and molybdenum, for example, platinum-tungsten, platinum-nickel or platinum-nickel-tungsten.

The foregoing metals may be incorporated into the catalyst by any suitable method suchs impregnation or exchange onto the zeolite. The metal may be incorporated in the form of a cationic, anionic or neutral complex such as $Pt(NH_3)_4^{2+}$ and cationic complexes of this type will be found convenient for exchanging metals onto the zeolite. Anionic complexes such as the vanadate or metatungstate ions are useful for impregnating metals into the zeolites. Incorporation is preferably undertaken in accordance with the method described in U.S. Patent No. 4,312,790.

The amount of the hydrogenation/dehydrogenation component is suitably from 0.01 to 10 percent by weight, normally 0.1 to 5 percent by weight, although this will, of course, vary with the nature of the component, less of the highly active noble metals, particularly platinum, being required than of the less active base metals.

The two components of the catalyst system of the invention differ from each other in two significant respects, namely their acidities and their xylene diffusion properties.

The acidity of a zeolite is conventionally expressed as its alpha value, a parameter which can be determined by the method described in the Journal of Catalysis, Vol. VI, page 278-287, 1966. The alpha value of the zeolite of the component (1) of the catalyst system which is effective to deethylate ethylbenzene to produce benzene and ethane/ethene, as well as to hydrocrack nonaromatic present, is at least 100, preferably 100 to 500 and more preferably 100 to 300. The alpha value of the zeolite of the component (2) of the catalyst system which is effective to isomerize the xylenes in the feed is less than 100, preferably, is less than or equal to 50 and most preferably is 5 to 25.

Each of the components of the catalyst system also exhibit mutually exclusive xylene diffusional properties. These properties can be identified by noting the time (in minutes) required to sorb 30% of the equilibrium capacity of ortho xylene at 120°C and at an o-xylene partial pressure of $600 \pm 100$ Pa ($4.5 \pm 0.8$ mm of mercury), a test described by Olson et al in U.S. Patent Nos. 4,117,026, 4,159,282 and Re. 31,782. Herein, that equilibrium capacity of ortho-xylene is defined as greater than 1 gram of xylene(s) per 100 grams of zeolite. In accordance with the invention, the deethylation component (1) has an o-xylene sorption time in excess of about 50 minutes and preferably greater than 100 but less than 10,000 minutes while the isomerization component (2) has an o-xylene sorption time less than 10 minutes. When the zeolite of deethylation component (1) is ZSM-5, the requisite xylene diffusion properties can be satisfied by providing the ZSM-5 as crystals having a minimum dimension of at least 1 $\mu$ (one micron). Production of ZSM-5 with minimum crystal dimensions of at least 1 $\mu$ is described in, for example, U.S. Patent No. 4,375,458, G.B. 1,581,513, and EP-A-0 026 962 and 0 026 963. Where the zeolite of the isomerization component (2) is also ZSM-5 the requisite xylene diffusion properties can be satisfied by providing the ZSM-5 as crystals having a minimum dimension less than 0.5 $\mu$. Preferably, that minimum dimension of ZSM-5 used as the isomerization component (2) is at most 0.10 $\mu$ ; and most preferably that minimum dimension ranges from 0.02 to 0.05 $\mu$.

In practicing the process of the invention, it may be desirable to formulate the catalyst system of the invention with another material resistant to the temperature and other conditions of the process. Such matrix materials include synthetic or naturally occurring substances as well as inorganic materials such as clay, silica and/or metal oxides. The latter may be either naturally occurring or in the form of gelatinous precipitates or gels including mixtures of silica and metal oxides. Naturally occurring clays, which can be composited with the zeolite include those of the montmorillonite and kaolin families, which families includes the sub-bentonites and the kaolins commonly known as Dixie, McNamee : Georgia and Florida clays or others in which the main mineral constituent is halloysite, kaolinite, dickite, nacrite or anauxite. Such clays can be used in a raw state as originally mined or initially subjected to calcination acid treatment or chemical modification.

In addition to the foregoing materials, the zeolite employed herein may be composited with a porous matrix material, such as alumina, silica-alumina, silica-magnesia, silica-zirconia, silica-thoria, silica-berylia, silica-titania as well as ternary compositions, such as silica-alumina-thoria, silica-alumina-zirconia, silica-alumina-magnesia and silica-magnesia-zirconia. The matrix may be in the form of a cogel. The relative proportions of zeolite component and inorganic oxide gel matrix may vary widely with the zeolite content ranging from between 1 to 99 percent by weight and more usually in the range of 5 to 80 percent by weight of the composite.

The invention will now be more particularly described with reference to the Examples and the accompanying drawings in which

Figure 1 is a graph of ethylbenzene (EB) conversion in weight percent plotted against hydrocarbon (HC) WHSV, for various catalysts ;

Figure 2 is a graph of $C_9$ conversion in weight percent plotted against WHSV.

Figure 3 is a graph of xylene gains, in weight percent, plotted against EB conversion in weight percent.

## EXAMPLES

### Catalyst Preparative Procedure

### 1. Zeolite Preparation

ZSM-5 with a crystal size having minimum dimensions ranging from 0.02 and 0.05 $\mu$ was produced by the following procedure. A sodium silicate solution was prepared by mixing 16 parts water and 27.7 parts sodium silicate (28.7 wt.% $SiO_2$, 8.9 wt.% $Na_2O$, 62.4 wt.% $H_2O$) followed by addition of 0.08 parts Daxad 27 (W.R. Grace Chemical Division). The solution was cooled to approximately 15°C.

An acid solution was prepared by adding 1 part aluminum sulfate (17.2 wt.% $Al_2O_3$) to 16.4 parts water followed by 2.4 parts sulfuric acid (93 wt.% $H_2SO_4$) and 1.2 parts NaCl.

These solutions were mixed in an agitated vessel while 3.9 parts of NaCl were added. The molar ratios expressed as oxides were as follows in the resultant mixture :

$SiO_2/Al_2O_3$ = 78.5

$Na_2O/Al_2O_3$ = 49.5

The gel was then heated to about 93°C, agitation was reduced and an organic solution containing 0.8 parts n-propyl bromide and 1.5 parts methyl ethyl ketone was added above the gel. After these organics were added, 2.3 parts of n-propyl amine was added to the organic phase above the gel. This mixture was held at about 93°C for 6 hours, then severe agitation was resumed. Crystallization was conducted at 93°-99°C until the gel was 80% crystallized, at which time temperature was increased to 150°-160°C. Unreacted organics were removed by flashing and the remaining contents cooled. The zeolite slurry product was diluted with 4-5 parts water per part slurry and 0.0002 parts of flocculent (Rohm and Haas, Primafloc C-7) per part slurry, and allowed to settle. Supernant liquid was drawn off. The settled solids were reslurried to the original volume of the preceeding step with water and 0.00005 parts of flocculent per part slurry. After settling, the aqueous phase was decanted. This was repeated until the sodium level of the zeolite was less than 0,10 wt.%. Then 0.1 parts ammonium nitrate per part slurry were added to the settled solids and the water from the previous decantation. The mixture was reslurried and the solids were allowed to settle. The washed zeolite solids were filtered identified as ZSM-5 by X-ray diffraction, and analyzed as having a $SiO_2/Al_2O_3$ mole ratio of 62.6 and a sodium content of 0.02 wt.% (dry basis). The minimum dimensions of the crystals of ZSM-5 produced thereby ranged from 0.02 to 0.05 μ.

ZSM-5 with a crystal size having minimum dimensions of at least 1 micron was produced as described previously.

## 2. Preparation for Catalyst A-D

Extrudate samples consisting of 1.5 mm (1/16 in.) diameter particles of 65 wt% Na-form ZSM-5/35 wt% binder alumina were processed by calcination at 540°C to convert the inert sodium form of the zeolite into the active hydrogen or acid form. The acid zeolite-containing extrudates were then impregnated with a platinum salt to the desired Pt loading. In a preferred embodiment this was accomplished by evacuating the air from the extrudate sample and replacing the air with $CO_2$. The $CO_2$ saturated samples were then contacted with an aqueous solution of $H_2PtCl_6$, containing the equivalent of 0.3 wt% Pt, whereafter the samples were dried at 120°C (250°F) and the calcined at 480°C (900°F) in air.

Catalyst A : The minimum dimension of the ZSM-5 crystal in this example was approximately 2.5 to 4 μ. The extrudate catalyst has about 200 alpha before Pt impregnation and the final catalyst contained 0.3 wt.% Pt.

Catalyst B : As catalyst A but the minimum dimension of the ZSM-5 crystal was approximately 1-2 μ. The extrudate catalyst has about 200 alpha before Pt impregnation.

Catalyst C : As catalyst A but the minimum dimension of the ZSM-5 crystal was 0.02 to 0.05 μ.

Catalyst D (comprative) : Equivalent of Catalyst A without platinum.

Catalysts A, B and C relate to component (1).

## 3. Preparation of Catalyst E

An aqueous solution of $[Pt(NH_3)_4]Cl_2$ containing the equivalent of 0.1 wt% Pt was added to a 50/50 mixture of NaZSM-5 (minimum dimension 0.02 to 0.05 μ) and binder alumina. This Pt-containing mixture was extruded to form 1.5 mm (1/16 in). diameter particles. The extruded material was then calcined in nitrogen at 540°C (1000°F), ammonium ion exchanged, and calcined in air at 540°C (1000°F). The calcined extrudate was then treated with steam at 552°C (1025°F) for 24 hours. The resultant catalyst had a value of about 10 alpha and was useful as component (2) of the catalyst System of the invention.

## EXAMPLE 1 (Comparative)

Catalyst A alone was charged into a fixed-bed reactor and was pretreated with $H_2$ at 1480 kPa (200 psig) and 482°C (900°F) for about 2 hours before the liquid feedstock was charged into the reactor. The feedstock tested consisted of 8-40% ethylbenzene (EB) and 0-3% $C_9$ paraffins with the balance meta-rich mixed xylenes. The results are shown in Table 1 from which it will be seen that catalyst A alone showed insufficient p-xylene conversion activity, even though high EB conversion and low xylene loss were achieved.

## TABLE 1

| | Feed | | | |
|---|---|---|---|---|
| Temperature, °F(°C) | | 800(427) | 800(427) | 800(427) |
| Pressure, PSIG(kPa) | | 200(1480) | 200(1480) | 400(2859) |
| WHSV | | 2.9 | 8.6 | 8.7 |
| $H_2$/HC Molar Ratio | | 2.9 | 3.0 | 3.0 |
| Time on Stream, Hrs. | | 3.5 | 9.5 | 16.0 |
| | | | | |
| EB Conversion, Wt % | | 99.6 | 99.6 | 99.0 |
| Xylene Loss, Wt % | | 13.9 | 4.8 | 13.9 |
| | | | | |
| Equilibrium Approach, % P-Xylene | | 62.7 | 49.4 | 54.0 |

Hydrocarbon Product Distribution (Wt.%)

| | Feed | | | |
|---|---|---|---|---|
| $C_1$-$C_4$ | | 14.0 | 12.8 | 17.1 |
| $C_5$-$C_7$ (non-aromatic) | | 1.9 | 2.6 | 10.5 |
| $C_8$ Naphthene | | 0.3 | 0.6 | 3.9 |
| $C_9$ Naphthene | | 0.0 | 0.1 | |
| Benzene | 0.1 | 20.9 | 24.0 | 13.8 |
| Toluene | 1.2 | 13.6 | 5.4 | 5.7 |
| EB | 41.2 | 0.2 | 0.3 | 0.4 |
| P-Xylene | 5.9 | 9.1 | 9.1 | 8.4 |
| M-Xylene | 38.6 | 27.9 | 32.6 | 28.6 |
| O-Xylene | 13.0 | 12.0 | 12.5 | 11.4 |
| $C_9$+ Arom. | | 0.3 | 0.1 | 0.2 |
| Total | 100.0 | 100.0 | 100.0 | 100.0 |

EXAMPLE 2

The process of Example 1 was repeated on three different chargestocks and with the catalyst system comprising 1/3 catalyst A as a top layer in the catalyst bed and 2/3 catalyst E as the remainder of the bed. Charge stock 1 represents an extracted feed which contains low (i.e. 8%) EB (ethylbenzene), about 9% p-xylene, 62% m-xylene and about 20% o-xylene. Charge stock 2 represents a non-extracted feed derived from reformate heart-cut which typically contains about 3% $C_9$ paraffins, 20% EB, 1% p-xylene, 52% m-xylene and 25% o-xylene.

Charge stock 3 represents a high EB feed typically obtained from pyrolysis gasoline which contains about 40% EB, 6% p-xylene, 40% m-xylene and 13% o-xylene. In each case conversion was effected at 427°C (800°F), 1480 kPa (200 psig), 9WHSV and 3/1 $H_2$/hydrocarbon ratio. The results are given in Table 2, which also lists the results obtained with comparative tests effected using catalyst E above.

TABLE 2

EFFECT OF VARIOUS FEEDS

| Charge Stock | 1 | 1 | 2 | 2 | 3 | 3 |
|---|---|---|---|---|---|---|
| Catalyst | A/E | E | A/E | E | A/E | E |
| | | | | | | |
| EB Conversion, Wt % | 76 | 30 | 85 | 30 | 85 | 40 |
| Xylene Loss, % | 1.1 | 1.0 | 0.5 | 1.0 | 3.5 | 5.4 |
| P-xylene Equil. Appr. | 103 | 103 | 102 | 103 | 103 | 104 |
| $C_9$ Conversion, Wt % | – | – | 77 | 20 | – | – |

EXAMPLE 3 (Comparative)

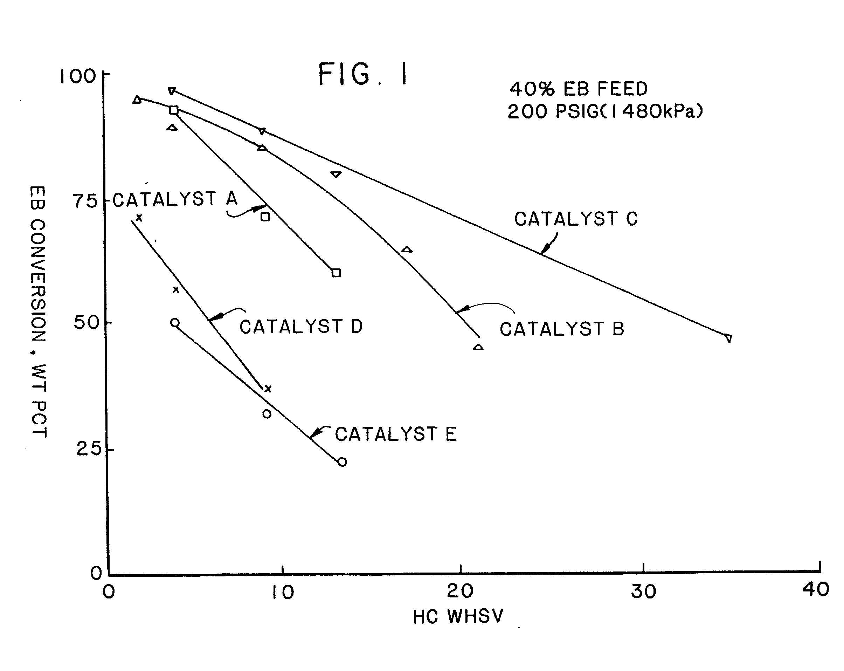

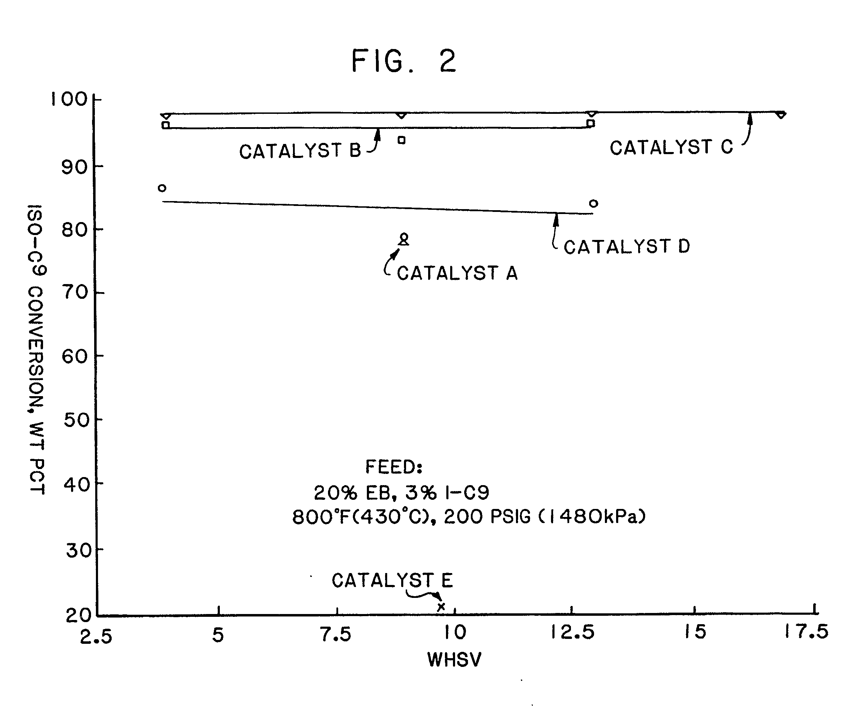

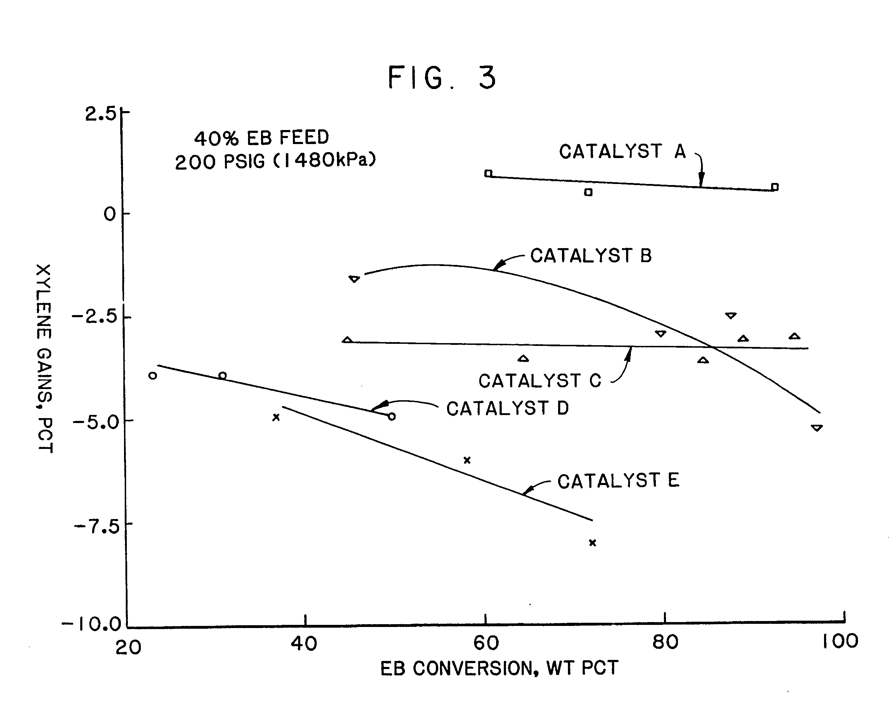

Catalysts A-E were evaluated individually for their EB conversion, iso-$C_9$ conversion and xylene loss and the results are shown in Figures 1-3 respectively. From these Figures it is clear that even though the smaller crystal size ZSM-5 (i.e. catalyst C) is more active than the larger crystal size ZSM-5 (i.e. catalysts A and B) in both EB and paraffin conversions (see Figures 1 and 2), xylene selectivity is however better for the larger crystal size ZSM-5 catalysts (see Figure 3).

**Claims**

1. A process for the treatment of a feed which contains an aromatic $C_8$ hydrocarbon mixture of ethylbenzene and xylenes, in which the concentration of para-xylene is less than that at thermal equilibrium, for dealkylating ethylbenzene and isomerizing xylenes to increase the concentration of para-xylene, which process comprises contacting the feed with a two component catalyst system including component (1) and component (2) wherein component (1) comprises a zeolite having a constraint index of 1 to 12 and an ortho-xylene sorption time of greater than 50 minutes based on its capacity to sorb 30% of the equilibrium capacity of ortho-xylene at 120°C and at an ortho xylene partial pressure of 600 ± 100 Pa (4.5 ± 0.8 mm of mercury) and 0.05 to 10 weight percent of a hydrogenation/dehydrogenation component ; and
component (2) comprises a zeolite, having a constraint index of 1 to 12 and a xylene sorption capacity greater than 1 gram/100 grams of zeolite and an ortho-xylene sorption time of less than 10 minutes based on its capacity to sorb 30% of the equilibrium sorption capacity of ortho-xylene at 120°C and an ortho-xylene partial pressure of 600 ± 100 Pa (4.5 ± 0.8 mm of mercury) and comprises 0.05 to 10 weight percent of a hydrogenation/dehydrogenation component, component (2) comprising at least 50 percent by volume of the catalyst system.
2. The process of claim 1 wherein the isomerization conditions include a temperature of 200-540°C (400 to 1000°F), a pressure of 100 to 7000 kPa, a WHSV of 0.5 to 100 and a $H_2$/HC molar ratio of 0.5 to 10.
3. The process of claim 1 or claim 2, wherein the feed is contacted with component (1) of the catalyst system before the feed is contacted with component (2) of the catalyst system.
4. The process of any preceding claim, wherein said hydrogenation/dehydrogenation component is platinum.
5. The process of any preceding Claim wherein said feed contains 0 to 20 weight percent non-aromatics and wherein component (1) is effective to hydrocrack said non-aromatics.
6. The process of any preceding Claim wherein the alpha value of the zeolite of component (1) is from 100 to 500.
7. The process of any preceding Claim wherein the alpha value of the zeolite of component (2) is less than 100.
8. The process of any preceding Claim wherein the alpha value of the zeolite of component (2) is less than 50.
9. The process of any preceding claim wherein the zeolite of component (1) is ZSM-5 having a crystal size with a minimum dimension greater than 1 micron.
10. The process of any preceding claim wherein the zeolite of component (2) is ZSM-5 having a crystal size with a minimum dimension less than 0.1 micron.

## Patentansprüche

1. Verfahren zur Behandlung einer Zufuhr, die eine aromatische C$_8$-Kohlenwasserstoffmischung von Ethylbenzol und Xylolen enthält, bei der die Konzentration von p-Xylol kleiner als die beim thermischen Gleichgewicht ist, zur Dealkylierung von Ethylbenzol und zur Isomerisierung von Xylolen, um die Konzentration an p-Xylol zu erhöhen, wobei dieses Verfahren den Kontakt der Zufuhr mit einem Zweikomponenten-Katalysatorsystem umfaßt, dr eine Komponente (1) und eine komponente (2) umfaßt, wobei

die Komponente (1) einen Zeolith, der einen Zwangsindex von 1 bis 12 und eine Sorptionszeit für o-Xylol von mehr als 50 Minuten aufweist, bezogen auf dessen Kapaztät, 30% der Gleichgewichtskapazität von o-Xylol bei 120°C und einem Partialdruck von o-Xylol von 600 ± 100 Pa (4,5 ± 0,8 mm Quecksilber) zu sorbieren, und 0,05 bis 10 Gew.-% einer Hydrierungs/Dehydrierungs-Komponente umfaßt, und

die Komponente (2) einen Zeolith, der einen Zwangsindex von 1 bis 12 und eine Sorptionskapazität für Xylol von mehr als 1 g/100 g des Zeolith und eine Sorptionszeit für o-Xylol von weniger als 10 Minuten aufweist, bezogen auf dessen Kapazität, 30% der Gleichgewichts-Sorptionskapazität von o-Xylol bei 120°C und einem Partialdruck von o-Xylol von 600 ± 100 Pa (4,5 ± 0,8 mm Quecksilber) zu sorbieren, und 0,05 bis 10 Gew.-% einer Hydrierungs/Dehydrierungs-Komponente umfaßt, wobei die Komponente (2) mindestens 50 Vol.-% des Katalysatorsystems umfaßt.

2. Verfahren nach Anspruch 1, worin die Isomerisierungsbedingungen eine Temperatur von 200 bis 540°C (400 bis 1000°F), einen Druck von 100 bis 7000 kPa, eine WHSV von 0,5 bis 100 und ein H$_2$/HC-Molverhältnis von 0,5 bis 10 umfassen.

3. Verfahren nach Anspruch 1 oder 2, worin die Zufuhr mit der Komponente (1) des Katalysatorsystems in Kontakt gebracht wird, ehe die Zufuhr mit der Komponente (2) dieses Katalysatorsystems in Kontakt gebracht wird.

4. Verfahren nach einem der vorstehenden Ansprüche, worin die Hydrierungs/Dehydrierungs-Komponente Platin ist.

5. Verfahren nach einem der vorstehenden Ansprüche, worin die Zufuhr 0 bis 20 Gew.-% Nichtaromaten enthält, und worin die Komponente (1) für das Hydrocracken der Nichtaromaten wirksam ist.

6. Verfahren nach einem der vorstehenden Ansprüche, worin der α-Wert der Zeolithkomponente (1) von 100 bis 500 beträgt.

7. Verfahren nach einem der vorstehenden Ansprüche, worin der α-Wert der Zeolithkomponente (2) weiniger als 100 beträgt.

8. Verfahren nach einem der vorstehenden Ansprüche, worin α-Wert der Zeolithkomponete (2) kleiner als 50 ist.

9. Verfahren nach einem der vorstehenden Ansprüche, worin der Zeolith der Komponente (1) ZSM-5 ist, der eine Kristallgröße mit einer Mindestabmessung von mehr als 1 Mikrometer aufweist.

10. Verfahren nach einem der vorstehenden Ansprüche, worin der Zeolith der Komponente (2) ZSM-5 ist, der eine Kristallgröße mit einer Mindestabmessung von weniger als 0,1 Mikrometer aufweist.

## Revendications

1. Un procédé pour le traitement d'une charge d'alimentation contenant un mélange d'hydrocarbures aromatiques en C$_8$ d'éthylbenzène et de xylènes, dans lequel la concentration du para-xylène est inférieure à la concentration à l'équilibre thermique, pour désalkyler l'éthylbenzène et isomériser des xylènes afin d'augmenter la concentration du para-xylène, caractérisé en ce qu'il comprend la mise en contact de la charge avec un système catalyseur à deux composants comprenant un composant (1) et un composant (2) dans lequel :

le composant (1) comprend une zéolite ayant un indice de contrainte de 1 à 12 et un temps de sorption de l'ortho-xylène supérieur à 50 minutes basé sur sa capacité de sorber 30% de la capacité de sorption à l'équilibre de l'ortho-xylène à 120°C et à une pression partielle d'ortho-xylène de 600 ± 100 Pa (4,5 ± 0,8 mm de mercure), et 0,05 à 10% en poids d'un composant d'hydrogénation/déshydrogénation ; et

le composant (2) comprend une zéolite ayant un indice de contrainte de 1 à 12 et une capacité de sorption de xylènes supérieure à 1 g/100 g de zéolité et un temps de sorption de l'ortho-xylène inférieur à 10 minutes basé sur sa capacité de sorber 30% de la capacité de sorption à l'équilibre de l'ortho-xylène à 120°C et à une pression partielle d'ortho-xylènede 600 ± 100 Pa (4,5 ± 0,8 mm de mercure), et 0,05 à 10% en poids d'un composant d'hydrogénation/déshydrogénation, le composant (2) représentant au moins 50% en volume du système catalyseur.

2. Un procédé suivant la revendication 1, caractérisé en ce que les conditions d'isomérisation comprennent une température de 200-540°C (400 à 1000°F), une pression de 100 à 7000 kPa, und VSHP de 0,5 à 100 et un rapport molaire $H_2$/HC de 0,5 à 10.

3. Un procédé suivant la revendication 1 ou la revendication 2, caractérisé en ce que la charge est mise en contact avec le composant (1) du système catalyseur avant d'être mise en contact avec le composant (2) du système catalyseur.

4. Un procédé suivant l'une quelconque des revendications précédentes, caractérisé en ce que le composant d'hydrogénation/déshydrogénation est le platine.

5. Un procédé suivant l'une quelconque des revendications précédentes, caractérisé en ce que cette charge contient de 0 à 20% en poids de composés non aromatiques et que le composant (1) est efficace pour hydrocraquer ces composés non aromatiques.

6. Un procédé suivant l'une quelconque des revendications précédentes, caractérisé en ce que la valeur alpha de la zéolite du composant (1) est de 100 à 500.

7. Un procédé suivant l'une quelconque des revendications précédentes, caractérisé en ce que la valeur alpha de la zéolite du composant (2) est inférieure à 100.

8. Un procédé suivant l'une quelconque des revendications précédentes, caractérisé en ce que la valeur alpha de la zéolite du composant (2) est inférieure à 50.

9. Un procédé suivant l'une quelconque des revendications précédentes, caractérisé en ce que la zéolite du composant (1) est une ZSM-5 ayant une dimension cristalline dont la dimension minimum est supérieure à 1 µm.

10. Un procédé suivant l'une quelconque des revendications précédentes, caractérisé en ce que la zéolite du composant (2) est une ZSM-5 ayant une dimension cristalline dont la dimension minimum est inférieure à 0,1 µm.

FIG. 1

40% EB FEED
200 PSIG(1480kPa)

CATALYST A
CATALYST C
CATALYST D
CATALYST B
CATALYST E

EB CONVERSION, WT PCT

HC WHSV

# FIG. 2

FEED:
20% EB, 3% I-C9
800°F(430°C), 200 PSIG (1480kPa)

Y-axis: ISO-C9 CONVERSION, WT PCT
X-axis: WHSV

CATALYST B
CATALYST C
CATALYST A
CATALYST D
CATALYST E

EP 0 234 684 B1

FIG. 3

40% EB FEED
200 PSIG (1480kPa)

CATALYST A

CATALYST B

CATALYST C

CATALYST D

CATALYST E

XYLENE GAINS, PCT

2.5
0
-2.5
-5.0
-7.5
-10.0

EB CONVERSION, WT PCT

20   40   60   80   100